# EUROPEAN PATENT APPLICATION

(11) **EP 1 215 212 A2**
(43) Date of publication of application: **19.06.2002**
(21) Application number: 01123067.9
(22) Date of filing: 30.12.1987
(51) Int. Cl.: C07H 15/203, C12P 19/00, C12R 1/29, C12P 19/44, A61K 31/70, C12N 1/20, A61P 31/04

(54) **Dihydro derivatives of LL-E33288 antibiotics**

(30) Priority: 30.01.1987 US 4154; 30.01.1987 US 4153; 30.01.1987 US 9321
(62) Divisional of application: 87119344.7
(71) Applicant: AMERICAN CYANAMID COMPANY, Wayne, NJ 07470-8426 (US)
(72) Inventor: Lee, May Dean-Ming, Monsey, New York 10952 (US); Greenstein, Michael, Suffern, New York 10901 (US); Labeda, David Paul, Peoria, Illinois 61614 (US); Fantini, Amedeo Alexander, New City, New York 10956 (US)
(74) Representative: Hartz, Nikolai F., Dr.

(57) **Abstract**

Antibacterial and antitumor agents designated pseudoaglycone and dihydro derivatives of LL-E33288 complex and their production by new strains of Micromonospora echinospora ssp. calichensis and NRRL-18149; and their preparation are disclosed.

## Description

This invention relates to new antibacterial and antitumor agents designated pseudoaglycone and dihydro derivatives of LL-E33288α₁-I, LL-E33288α₂-I, LL-E33288α₃-I, LL-E33288β₁-I, LL-E33288β₂-I, LL-E33288γ₁-I, and LL-E33288δ₁-I, LL-E33288α₁-Br, LL-E33288α₂-Br, LL-E33288α₃-Br, LL-E33288β₁-Br, LL-E33288β₂-Br LL-E33288γ₁-Br and LL-E33288δ₁-Br to their production by fermentation, to methods for their recovery and concentration from crude solutions and to processes for their purification. The present invention includes within its scope the anti-bacterial and antitumor agents in dilute form, as crude concentrates, as a complex of various or all components, in pure form as individual components and novel strains of Micromonospora.

The LL-E33288 antibiotics are closely related compounds. The fourteen antibiotics are recovered from fermentation and are initially obtained as a mixture, hereinafter either the LL-E33288 complex, the LL-E33288 Iodo-complex or the LL-E33288 Bromo-complex. In general, the iodine containing components of the LL-E33288 antibiotics (e.g., α₁-I, α₂-I, α₃-I, β₁-I, β₂-I, γ₁-I, and δ₁-I) are found only in fermentation using media containing inorganic or organic iodide while the bromine containing components (e.g., α₁-Br, α₂-Br, α₃-Br, α₄-Br, β₁-Br, β₂-Br, and γ₁-Br) are found only in fermentations using media containing inorganic or organic bromide. While the ratio of components in the LL-E33288 complex will vary, depending upon the fermentation of both the bromine and the iodine containing antibiotics, LL-E33288β₁, LL-E33288γ₁ and LL-E33288δ₁-I, are generally the major components, together accounting for approximately 90% of the complex. LL-E33288α₁, LL-E33288α₂, LL-E33288α₃, LL-E33288α₄ and LL-E33288β₂ are minor components, together accounting for approximately 10% of the complex.

The LL-E33288 antibiotics are active against gram-positive and gram-negative bacteria. Each of the components were also found to be active in a modification of the Biochemical Induction Assay [Elespuru, R. and Yarmolinsky, M., Environmental Mutagenesis, 1, 65-78 (1978)], a test which specifically measures the ability of an agent to directly or indirectly initiate DNA damage. In this assay, both LL-E33288γ₁-I and LL-E33288δ₁-I were active at concentrations lower than 1x10⁻⁶ mcg/ml.

### DETAILED DESCRIPTION OF THE INVENTION

Proposed structures of some of the LL-E33288 antibiotics are disclosed below.

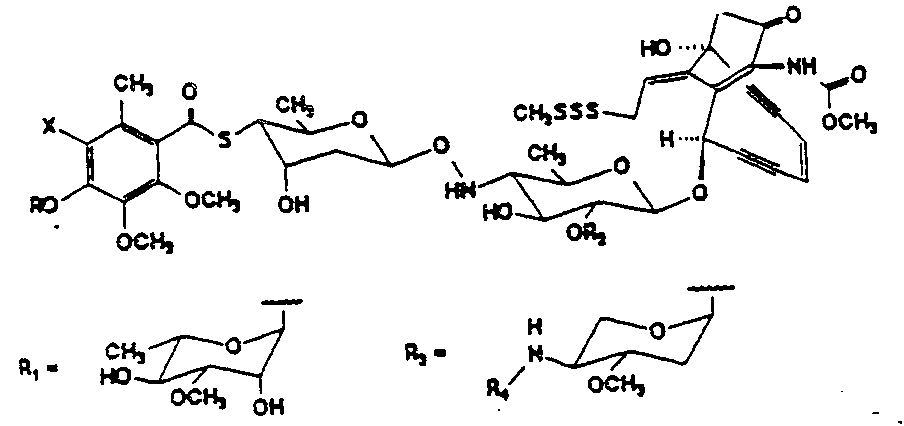

| E33288 | X | R | R₂ | R₄ |
|---|---|---|---|---|
| α₂^{I} | I | H | R₃ | C₂H₅ |
| α₃^{I} | I | R₁ | H | |
| β₁^{I} | I | R₁ | R₃ | (CH₃)₂CH |
| γ₁^{I} | I | R₁ | R₃ | C₂H₅ |
| δ₁^{I} | I | R₁ | R₃ | CH₃ |
| β₁^{Br} | Br | R₁ | R₃ | (CH₃)₂CH |
| γ₁^{Br} | Br | R₁ | R₃ | C₂H₅ |
| α₂^{Br} | Br | H | R₃ | C₂H₅ |
| α₃^{Br} | Br | R₁ | H | |

The dihydro-LL-E33288-pseudoaglycone has the following proposed structure: 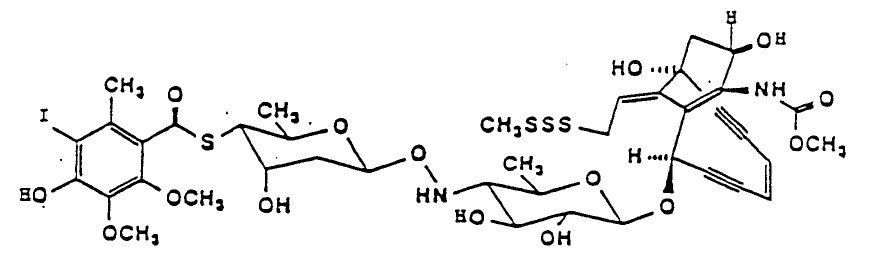 and the following physico-chemical characteristics:
a) molecular weight: 1052, determined by FAB-MS;
b) ultraviolet absorption spectrum: as shown in Figure I (methanol);
c) infrared absorption spectrum: as shown in Figure II (KBr disc);
d) proton magnetic resonance spectrum: as shown in Figure III (300MHz, CDCl₃); and
e) carbon-13 magnetic resonance spectrum: as shown in Figure IV (75MHz, CDCl₃).

When a dilute methanolic solution of an iodinated LL-E33288 component such as LL-E33288γ₁-I is treated with a cation exchange resin such as Dowex(R) 50W-X8(H form) the biologically active pseudoaglycone, having the following physico-chemical characteristics and proposed structure is obtained.
a) Molecular weight: 1050, determined by FAB-MS;
b) Molecular formula: C₄₀H₄₇N₂O₁₅IS₄, exact mass for M+Na was determined by high resolution FAB-MS t be 1073.0810 for C₄₀H₄₇N₂O₁₅IS₄Na;
c) Ultraviolet absorption spectra: as shown in Figure V (methanol; 0.1N HCl; o.1N NaOH);
d) Infrared absorption spectrum: as shown in Figure VI (KBr disc);
e) Proton magnetic resonance spectrum: as shown in Figure VII (300MHz, CDCl₃); and
f) Carbon-13 magnetic resonance spectrum: as shown in Figure VIII (75.43MHz, CDCl₃, ppm from TMS) significant peaks as listed in Table I.

**TABLE I**

| Peak No. | PPM | Peak No. | PPM |
|---|---|---|---|
| 1 | 17.8 q | 21 | 84.4 s |
| 2 | 19.1 q | 22 | 87.5 s |
| 3 | 22.8 q | 23 | 98.7 s |
| 4 | 24.7 q | 24 | 99.7 d |
| 5 | 36.8 t | 25 | 100.4 s |
| 6 | 39.1 t | 26 | 103.5 d |
| 7 | 51.6 d | 27 | 124.1 d |
| 8 | 53.3 t | 28 | 124.2 d |
| 9 | 53.6 q | 29 | 126.8 s |
| 10 | 61.0 q | 30 | 127.5 d |
| 11 | 61.5 q | 31 | 130.6 s |
| 12 | 67.2 d | 32 | 133.2 s |
| 13 | 68.2 d | 33 | 136.3 s |
| 14 | 69.1 d | 34 | 136.4 s |
| 15 | 69.6 d | 35 | 140.7 s |
| 16 | 70.1 d | 36 | 148.8 s |
| 17 | 71.3 d | 37 | 150.9 s |
| 18 | 72.5 s | 38 | 154.3 s |
| 19 | 74.5 d | 39 | 191.8 s |
| 20 | 83.9 s | 40 | 192.0 s |

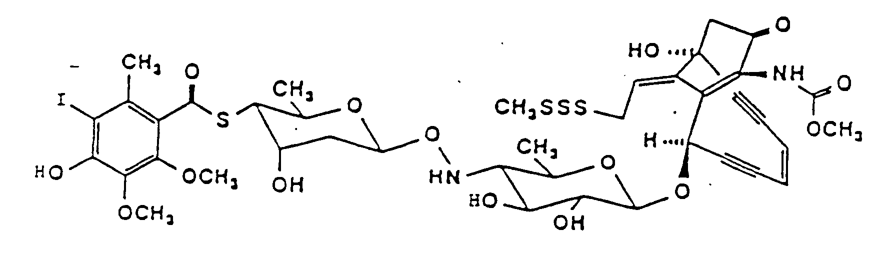

When the pseudoaglycone of LL-33288 is reacted with triphenylphosphine in a mixture of dichloromethane and methanol, the antibacterially inactive compound having the following physico-chemical characteristics and proposed structure is obtained.
a) Molecular weight: 974, determined by FAB-MS;
b) Molecular formula: C₃₉H₄₇N₂O₁₅IS₄, exact mass for M+Na was determined by high resolution FAB-MS to be 997.1370 for C₃₉H₄₇N₂O₁₅IS₂Na;
c) Infrared absorption spectrum: as shown in Figure IX (methanol: 0.1N HCl; 0.1N NaOH);
d) Infrared absorption spectrum: as shown in Figure X (KBr disc);
e) Proton magnetic resonance spectrum: as shown in Figure XI (300MHz, CDCl₃); and
f) Carbon-13 magnetic resonance spectrum: as shown in Figure XII (75.4MHz, CDCl₃, ppm form TMS) significant peaks as listed as follows:

| Peak No. | PPM | Peak No. | PPM |
|---|---|---|---|
| 1 | 17.8 q | 21 | 80.1 d |
| 2 | 19.1 q | 22 | 84.3 d |
| 4 | 24.7 q | 23 | 99.7 d |
| 5 | 36.7 t | 24 | 104.8 d |
| 6 | 38.5 t | 25 | 122.5 d |
| 7 | 51.9 q | 26 | 124.1 d |
| 8 | 52.8 q | 27 | 126.9 d |
| 9 | 56.6 t | 28 | 128.8 d |
| 10 | 61.1 q | 29 | 130.7 d |
| 11 | 61.5 q | 30 | 131.3 d |
| 12 | 67.2 d | 31 | 132.3 s |
| 13 | 68.3 d | 32 | 133.2 s |
| 14 | 69.0 d | 33 | 136.5 s |
| 15 | 69.2 d | 34 | 140.3 s |
| 16 | 69.2 d | 35 | 143.3 s |
| 17 | 70.1 d | 36 | 148.9 s |
| 18 | 73.0 s | 37 | 150.9 s |
| 19 | 73.5 s | 38 | 157.1 s |
| 20 | 75.2 d | 39 | 191.8 s |
| | | 40 | 199.8 s |

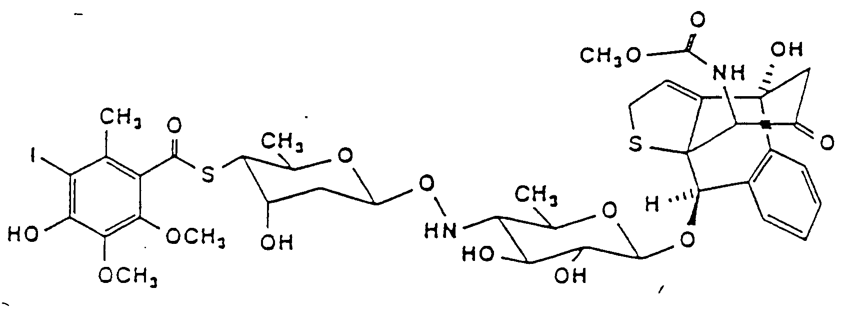

When the product derived from reacting the pseudoaglycone of LL-E33288 with triphenylphosphine in a mixture of dichloromethane and methanol is reacted first with a methanolic solution of potassium carbonate and then with excess acetic anhydride, the compound having the following physico-chemical characteristics and structure is obtained. This compound is crystalline and its chemical structure was determined by X-ray crystallography.
a) Ultraviolet absorption spectra: as shown in Figure XIII (methanol; 0.1N HCl: 0.1N NaOH) ;
b) infrared absorption spectrum: as shown in Figure XIV (KBr disc);
c) Proton magnetic resonance spectrum: as shown in Figure XV (300MHz, CDCl₃, containing trace ethyl acetate);
d) Molecular formula: C₃₆H₄₀NO₁₃IS₂, calculated from the chemical structure.

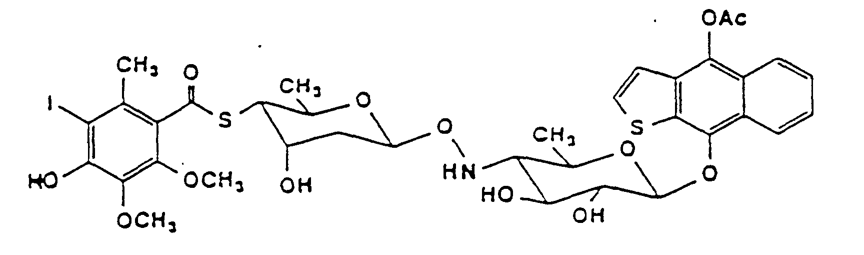

It has now been discovered that all of the aforementioned LL-E33288 components produced by NRRL-15839 and 15975 are also produced by a newly derived mutant named LL-E33288-UV 784, in much higher yields.

### Derivation of Mutant LL-E33288-UV 784

Vegetative growth from isolate UV 610(3) (see following diagram) was prepared as employed for fermentation and used to inoculate a flask of medium consisting of peptone, dextrose, molasses and water. The medium was supplemented with LL-E33288β₁-Br at a concentration of about 8 µg/ml. A number of platings were done from this flask and a resistant population was obtained on the seventh day. A total of 97 colonies (R1 to R97) were isolated. Isolate R66 became NRRL-15975. Isolate R80 is essentially similar to R66 in its biosynthetic potential.

Isolate R80 was then used as the starting culture from which a spore suspension was prepared and exposed to relatively high concentrations of the LL-E33288 complex, the purpose being to obtain isolates resistant to the LL-E33288 antibiotics and thereby improve production yields.

One survivor, labeled T₂ did produce greater yields of LL-E33288β₁-Br and LL-E33288γ₁-I in flask fermentations.

A spore suspension of T₂ was prepared and exposed to UV-irradiation. A total of 131 colonies were then isolated (UV 703 to UV 834), fermented and assayed. From this group, isolate UV 784 was selected for its activity in flask fermentations. Isolate UV 784, when fermented in the iodine containing medium, produced approximately double the yield of R66 (NRRL-15975).

Diagram of UV 784 Derivation
E-33288 (Wild type)(NRRL-15839)
Natural selection
NS6
Ultraviolet radiation
UV610
Natural selection
UV610(3)
Exposure to LL-E33288β1-Br
R80
R66 (NRRL-15975)
Exposure to LL-E33288 complex
T2
Ultraviolet radiation
UV 784

The mutant LL-E33288 UV 784 is maintained by that number in the culture collection of the Medical Research Division, American Cyanamid Company, Pearl River, New York. A viable culture of this microorganism has been deposited with the Culture Collection Laboratory, Northern Regional Research Center, U. S. Department of Agriculture, Peoria, Illinois on December 3, 1986, and has been added to its permanent collection. Access to said culture, under strain designation NRRL-18149, during pendency of the instant application shall be available to one determined by the Commissioner of Patents and Trademarks to be entitled thereto under C.F.R. §1.14 and 35 U.S.C. §122, and all restrictions on availability to the public of such culture will be irrevocably removed upon grant of a U. S. Patent on the instant application.

It is to be understood that for the production of these new antibacterial and anti-tumor agents the present invention is not limited to this particular organisms or to organisms fully answering the above growth microscopic characteristics which were given for illustrative purposes only. In fact, it is desired and intended to include the use of mutants produced from these organisms by various means such as exposure to X-radiation, ultraviolet radiation, N'-methyl-N-nitro-N-nitrosoquanidine, actinophages and the like.

The in vitro antibacterial activity of dihydro LL-E33288gamma l-I was determined against a spectrum of gram-positive and gram-negative bacteria by a standard agar dilution method. Mueller-Hinton agar containing two-fold decreasing concentrations of the antibiotics was poured into petri plates. The agar surfaces were inoculated with 1 to 5 x 10⁴ colony forming units of bacteria by means of a Steers replicating device. The lowest concentration of LL-E33288 component that inhibited growth of a bacterial strain after about 18 hours of incubation at approximately 35°C was recorded as the minimal inhibitory concentration (MIC) for that strain. The results are summarized in Table II.

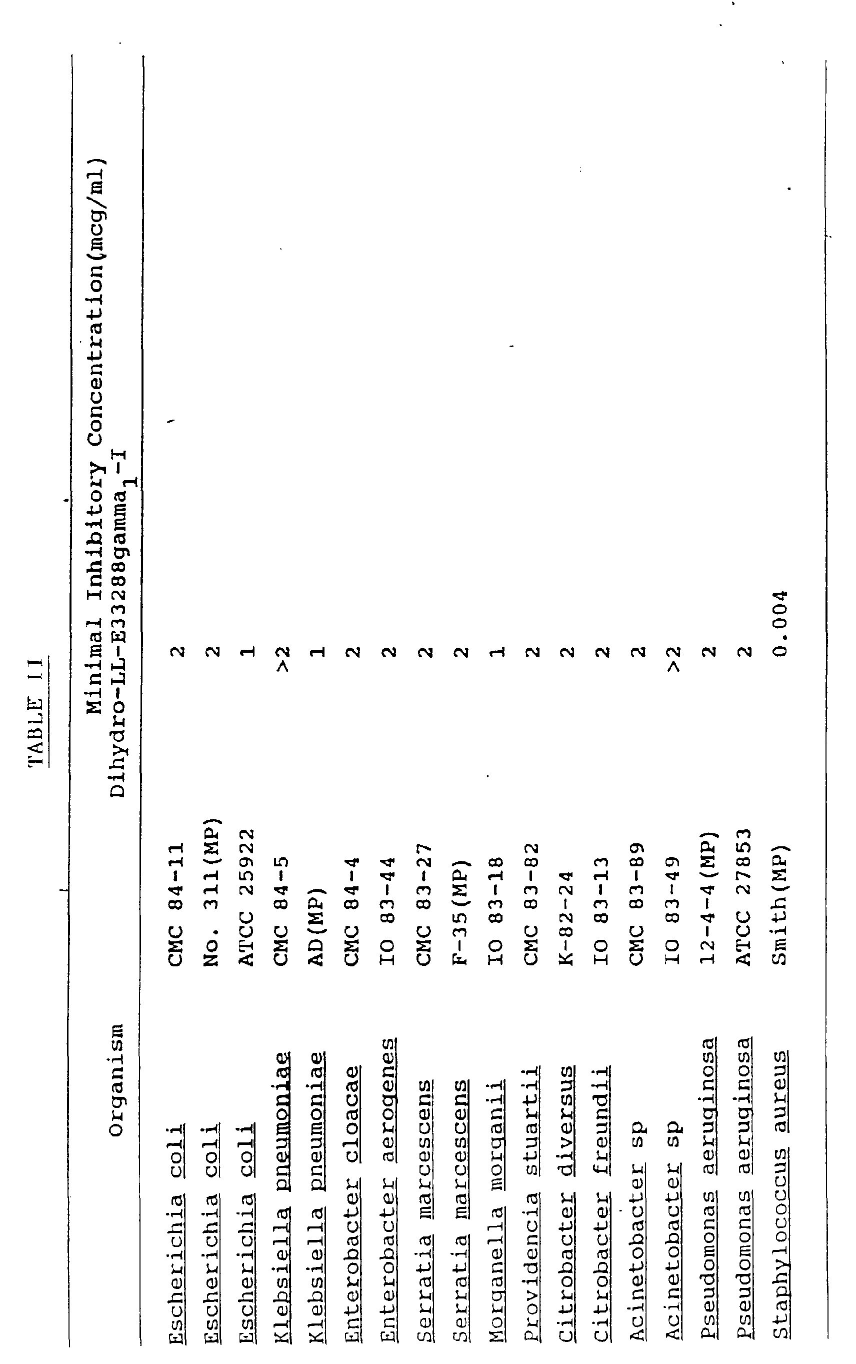

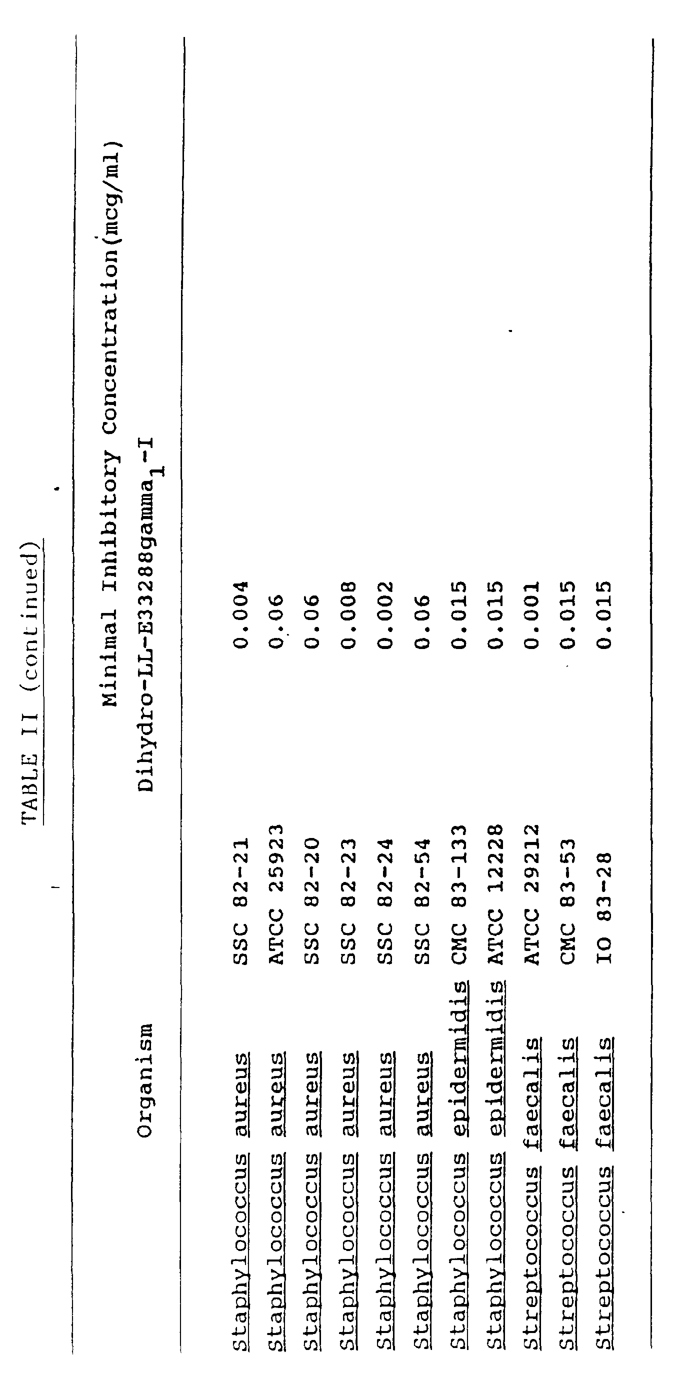

### General Fermentation Conditions

Cultivation of Micromonospora echinospora NRRL-15839 or NRRL-15975 may be carried out in a wide variety of liquid culture media. Media which are useful for the production of these novel antibacterial and anti-tumor agents include an assimilable source of carbon, such as starch, sugar, molasses, glycerol, etc.; an assimilable source of nitrogen such as protein, protein hydrolysate, polypeptides, amino acids, corn steep liquor, etc.; and inorganic anions and cations, such as potassium, sodium, ammonium, calcium, sulfate, carbonate, phosphate, chloride, etc. and sources of either bromine (sodium bromide) or iodine (potassium iodide). Trace elements such as boron, molybdenum, copper, etc., are supplied as impurities of other constituents of the media. Aeration in tanks and bottles is supplied by forcing sterile air through or onto the surface of the fermenting medium. Further agitation in tanks is provided by a mechanical impeller. An antifoam agent such as silicone may be added as needed.

### General Procedure for the Isolation and Separation of the Antibiotics - LL-E33288

The LL-E33288 antibiotics are recovered from the fermentation broth by extracting the whole mash with an organic solvent such as ethyl acetate or dichloromethane. The antibiotic complex, contained in the organic extract, is further purified by selective precipitation from lower hydrocarbons. The crude LL-E33288 antibiotic complex thus obtained is further purified and separated into the individual components by a series of column chromatographies using silica gel, Sephadex® LH-20 (Pharmacia Fine Chemicals) and C₁₈ bonded silica.

The invention will be described in greater detail in conjunction with the following non-limiting specific examples.

### Example 1

### Small Scale Preparation of LL-E33288 Complex from LL-E33288-UV 784, NRRL-18149

A suspension containing spores and mycelia was prepared from a slant of Micromonospora echinospora ssp. calichensis, LL-E33288-UV 784 (NRRL-18149) by adding 5-8 ml of water and scraping the surface of the slant. This suspension was used to inoculate 50 ml of a sterile medium of the following composition:

| | |
|---|---|
| Yeast extract | 0.5% |
| Beef extract | 0.3% |
| Tryptose | 0.5% |
| Dextrin | 2.4% |
| Dextrose | 0.5% |
| Calcium carbonate | 0.4% |
| Water qs | 100% |

The above medium, in a 250 ml Erlenmeyer flask, was incubated at 28°C on a rotary shaker at 200 rpm for 3-4 days thus providing stage I inoculum.

Stage I inoculum was used to inoculate 50 ml of the same sterile medium in a 250 ml baffled flask and incubated at 28°C on a rotary shaker at 250 rpm for 2 days, thus providing stage II inoculum.

Stage II inoculum was used to inoculate 100 ml of sterile fermentation medium of the following composition:

| | |
|---|---|
| Sucrose | 2.0% |
| Ferrous sulfate heptahydrate | 0.01% |
| Magnesium sulfate heptahydrate | 0.02% |
| Calcium carbonate | 0.25% |
| Peptone | 0.4% |
| Molasses | 0.25% |
| Potassium iodide* | 0.01% |
| Water.....qs | 100% |

| | |
|---|---|
| *Potassium bromide may be substituted to produce the bromo analogs. | |

The above medium in 500 ml baffled flasks was incubated at 28-30°C on a rotary shaker at 250 rpm for 6 days at which time the fermentation was harvested.

### Example 2

### Large Scale Fermentation of LL-E33288 Complex Using LL-E33288-UV 784

A three stage inoculum was prepared using a culture of LL-E33288-UV 784 (NRRL-18149). The inoculum media were of the following formulation:

| Ingredient | per/liter |
|---|---|
| Calcium carbonate | 4 g |
| Hodag® FD 82* | 1 ml |
| Dextrin | 24 g |
| Glucose | 5 g |
| Yeast extract | 5 g |
| Tryptone | 5 g |
| Beef extract | 3 g |
| Water qs | |

| | |
|---|---|
| *Hodag® FD 82 is a silicone antifoam agent. | |

The first stage consisted of 100 ml of the above sterile medium in a 500 ml flask incubated at 32°C and 200 rpm for 2 days. This first stage was used to inoculate a second stage consisting of 10 liters of the above sterile medium which was grown for 2 days at 32°C and 450 rpm in a small fermenter with a sterile air flow of one volume of air per volume of mash per minute (VVM). This second stage was used to inoculate a third stage consisting of 300 liters of the above sterile medium which was grown for 2 days at 32°C, 200-250 rpm and a sterile air flow of 0.67 VVM in a tank fermenter.

A 150 liter portion of this stage III inoculum was used to inoculate a sterile 1500 liter fermentation medium of the following composition:

| Ingredient | per/liter |
|---|---|
| Sucrose | 20.0 g |
| Ferrous sulfate heptahydrate | 0.1 g |
| Magnesium sulfate heptahydrate | 0.2 g |
| Peptone | 5.0 g |
| Molasses | 5.0 g |
| Potassium iodide* | 0.5 g |
| Calcium carbonate | 5.0 g |
| Hodag® FD 82 | 5.0 ml |
| Water qs | |

| | |
|---|---|
| *Potassium bromide may be substituted to produce the bromo analogs. | |

The fermentation was carried out at 30°C, a sterile air flow of 0.75 VVM, a back pressure of 8 psig and agitation by an impeller driven at 120 rpm for 5-6 days at which time the mash was harvested.

### Example 3

### Isolation of Crude LL-E33288α₃-I,LL-E33288β₁-I, LL-E33288γ₁-I and LL-E33288δ₁-I from a Fermentation of LL-E33288-UV-784 (NRRL-18149)

A 1500 liter portion of whole harvest mash containing 12.4 g of LL-E33288γ₁-I and 10.5 g of LL-E33288δ₁-I, which had been conducted essentially as described in Example 12, was mixed thoroughly with 1500 liters of ethyl acetate for 3 hours, then filter aid was added and the mixture filtered. The organic phase was separated, concentrated to 100 liters, adjusted to pH 6-7 with 2N sodium hydroxide and any aqueous phase discarded. The organic phase was further concentrated to 20 liters and any aqueous phase and interfacial fats removed. The organic phase was finally concentrated to a golden yellow syrup which was poured slowly into 7-8 times its volume of rapidly stirred hexane. The hexane insoluble gum, containing the LL-E33288 antibiotics, was collected, redissolved in 3 liters of ethyl acetate and dried over anhydrous sodium sulfate. The dried ethyl acetate solution was concentrated to a small volume and precipitated by the addition of ether and hexane, giving 53 g of crude LL-E33288 complex, containing 4.9 g of γ₁-I, 2.8 g of δ₁-I and small amounts of α₃-I and β₁-I.

### Example 4

### Separation of LL-E33288β₁-I, γ₁-I, δ₁-I and α₃-I

A 7.2 g portion of crude LL-E33288 complex from Example 13 was chromatographed on two Sepralyte C-18 (35-65 m) columns (2.5 x 23 cm), eluting with acetonitrile:0.2 M aqueous ammonium acetate (45:55) at 12 ml/minute, collecting sixty 24 ml fractions from each column. Each fraction was analyzed by TLC (EM silica gel 60F₂₅₄ pre-coated aluminum sheets, 3% isopropanol in ethyl acetate saturated with 0.1 M KH₂PO₄ elution, detected with UV₂₅₄ₙₘ quenching and bioautography via the BIA) and those containing γ₁-I were pooled and concentrated on a rotary evaporator to remove acetonitrile. The aqueous mixture was extracted twice with equal volumes of ethyl acetate and the ethyl acetate solution dried over anhydrous sodium sulfate, concentrated and precipitated by addition of hexane to yield 504 mg of partially purified LL-E33288γ₁-I (60% pure) containing β₁-I.

Fractions containing α₃-I and δ₁-I eluting off the column ahead of γ₁-I, were pooled separately and worked up to yield 812 mg of partially purified α₃-I (12% pure) and 1336 mg (22% δ₁-I, 20% γ₁-I) of a partially purified mixture of δ₁-I and γ₁-I.

### Example 5

### Purification of LL-E33288γ₁-I

A 309 mg portion of partially purified γ₁-I (66% pure) was chromatographed on a Sephadex® LH-20 (hydroxy propylated dextran) column (1.5 x 90 cm) equilibrated with hexane:dichloromethane:ethanol (2:1:1). The column was eluted with the same solvent system at 1.5 ml/minute and twenty-five 20 ml fractions were collected and analyzed as before. Fractions containing pure γ₁-I were pooled and concentrated as before to a light yellow residue. This residue was redissolved in ethyl acetate and precipitated by the addition of hexane to yield 194 mg of pure LL-E33288γ₁-I.

### Example 6

### Purification of LL-E33288β₁-I

A 1.05 g portion of partially purified γ₁-I containing β₁-I (61% γ₁-I, 10% β₁-I) was chromatographed on a Woelm silica (32-63 µ) column (1.5 x 45 cm) packed and equilibrated with ethyl acetate. The column was eluted with ethyl acetate at 3.6 ml/minute for one hour, then the eluent was changed to ethyl acetate:methanol (97:3) and the elution was continued for 2 hours. Fractions of 18 ml were collected during the entire elution. Each fraction was analyzed as before and those containing β₁-I were pooled and worked up to yield 56 mg of 86% pure LL-E33288β₁-I.

Fractions containing γ₁-I were also worked up to yield 385 mg of 74% pure LL-E33288γ₁-I.

### Example 7

### Purification of LL-E33288δ₁-I

A partially purified mixture of δ₁-I and γ₁-I (1.8 g, containing 648 mg of γ₁-I and 540 mg of δ₁-I) was chromatographed on a Woelm silica (32-63 µ) column (1.5 x 45 cm) packed and equilibrated with ethyl acetate. The column was eluted with ethyl acetate at 3 ml/minute for one hour, than the eluent was changed to ethyl acetate:methanol (97:3) and the elution was continued for 2 hours. Fractions of 15 ml were collected during the entire elution. Each fraction was analyzed as before and those containing pure δ₁-I were pooled and worked up to yield 367 mg of pure LL-E33288δ₁-I.

Fractions containing γ₁-I were also worked up to yield 574 mg of 65% pure LL-E33288γ₁-I.

### Example 8

### Purification of LL-E33288α₃-I

A partially purified sample of α₃-I (1.8 g, containing 310 mg of α₃-I) was chromatographed on a Sephadex® LH-20 column (1.5 x 90 cm) equilibrated with hexane:dichloromethane:ethanol (2:1:1). The column was eluted with the same solvent system at 4 ml/minute and forty-five 20 ml fractions were collected and analyzed as before. Fractions containing pure α₃-I were pooled and concentrated as before to a light yellow residue which was redissolved in ethyl acetate and precipitated by the addition of hexane to yield 289 mg of pure LL-E33288α₃-I.

### Example 9

### Preparation of LL-E33288α₂-I from LL-E33288γ₁-I

A 300 mg portion of partially purified γ₁-I (60% pure) was dissolved in 60 ml of 2% hydrogen chloride in methanol and the solution was allowed to remain at room temperature for 6 hours. The reaction mixture was then neutralized by the addition of a saturated methanolic solution of potassium carbonate. The precipitated potassium chloride was filtered off and the solution was concentrated to dryness. The ethyl acetate soluble portion of the residue was concentrated and precipitated from hexane to yield 135 mg of crude LL-E33288α₂-I.

This crude α₂-I was purified by chromatography on a Bio-Sil® (20-40 µ) column (1.5 x 20 cm) eluting with dichloromethane:methanol (96:4) to give 34 mg of analytically pure LL-E33288α₂-I. The small amounts of LL-E33288α₂-I isolated previously from the fermentation of NRRL-15839 was identified with the LL-E33288α₂-I prepared as described in this example by TLC and HPLC analyses.

Degradation products, termed pseudaglycones, of LL-E33288, BBM-1675, FR-900405, FR-900406, PD 114759, PD 115028, CL-1577A, CL-1577B, CL-1577D, CL-1577E and CL-1724 antibiotics/antitumor agents are disclosed and described.

Certain other antibiotics are pertinent to this invention, namely:
1) Esperamicin BBM-1675, a novel class of potent anti-tumor antibiotics. I. Physico-chemical data and partial structure. M. Konishi, et al., J. Antibiotics, 38, 1605 (1985). A new antitumor antibiotic complex. M. Konishi, et al., UK Patent application GB 2,141,425A, May 15, 1984.
2) New antitumor antibiotics, FR-900405 and FR-900406. I. Taxonomy of the producing strain. M. Iwami, et al., J. Antibiotics, 38, 835 (1985). New antitumor antibiotics FR-900405 and FR-900406. II. Production, isolation, characterization and antitumor activity. S. Kiyoto, et al., J. Antibiotics, 38, 840 (1985).
3) PD 114759 and PD 115028, novel antitumor antibiotics with phenomenal potency. I. Isolation and characterization. R. H. Bunge, et al., J. Antibiotics, 37, 1566 (1984). Biological and biochemical activities of the novel antitumor antibiotic PD 114759 and related derivatives. D. W. Fry, et al., Investigational New Drugs, 4, 3 (1986).
4) New antibiotic complex CL-1577A and CL-1577B produced by *Streptomyces* sp. ATCC 39363. European Patent application 0,132,082,A2.
5) CL-1577D and CL-1577E Antibiotic antitumor compounds, their production and use. U. S. Patent 4,539,203.
6) CL-1724 Antibiotic compounds, their production and use. U. S. Patent 4,554,162.

All of the information regarding BBM-1675, FR-900405, FR-900406, PD 114759, PD 115028, CL-1577A, CL-1577B, CL-1577D, CL-1577E and CL-1724 contained in the above citations is incorporated herein by reference.

This invention is also concerned with degradation products of the LL-E33288 antibiotics as well as degradation products of the BBM-1675, FR-900405, FR-900406, PD 114759, PD 115028, CL-1577A, CL-1577B, CL-1577D, CL-1577E and CL-1724 antibiotics referred to in the background of the invention, all of which are derived in the same manner and all of which are described as pseudoaglycones.

These pseudoaglycones are all active as anti-bacterial and antitumor agents and are derived by the same general procedure. For simplicity the procedure and activity will be described with reference to the iodinated LL-E33488 antibiotics. However, it is apparent to one skilled in the art that similar procedure and activity can be described for other modification of the antibiotics, e.g., the brominated LL-E33288 antibiotics.

The pseudoaglycone of LL-E33288 is active as an antibacterial agent when tested by the standard agar dilution method. This activity was determined against a spectrum of gram-positive and gram-negative bacteria. Mueller-Hinton agar containing two-fold decreasing concentrations of pseudoaglycone was poured into petri plates. The agar surface was inoculated with 1 to 5 x 10⁶ colony-forming units of bacteria by means of the Steers replicating device. The lowest concentration of the pseudoaglycone in mcg/ml that inhibited growth of a bacterial strain after about 18 hours of incubation at approximately 35°C was recorded as the minimal inhibitory concentration (MIC) for that strain. The results appear in Table III.

**TABLE III**

| Organism | | Minimal Inhibitory Concentration (mcg/ml) |
|---|---|---|
| Escherichia coli | CMC 84-11 | 2 |
| Escherichia coli | No. 311(MP) | 1 |
| Escherichia coli | ATCC 25922 | 1 |
| Klebsiella pneumoniae | CMC 84-5 | 2 |
| Klebsiella pneumoniae | AD(MP) | 0.5 |
| Enterobacter cloacae | CMC 84-4 | 4 |
| Enterobacter aerogenes | IO 83-44 | 4. |
| Serratia marcescens | CMC 83-27 | 1 |
| Serratia marcescens | F-35(MP) | 2 |
| Morganella morganii | IO 83-18 | 1 |
| Providencia stuartii | CMC 83-82 | 2 |
| Citrobacter diversus | K-82-24 | 2 |
| Citrobacter freundii | IO 83-13 | 1 |
| Acinetobacter sp. | CMC 83-89 | 2 |
| Acinetobacter sp | IO 83-49 | 4 |
| Pseudomonas aeruginosa | 12-4-4(MP) | 2 |
| Pseudomonas aeruginosa | ATCC 27853 | 1 |
| Staphylococcus aureus | Smith(MP) | 0.03 |
| Staphylococcus aureus | SSC 82-21 | 0.12 |
| Staphylococcus aureus | ATCC 25923 | 0.25 |
| Staphylococcus aureus | SSC 82-20 | 0.25 |
| Staphylococcus aureus | SSC 82-23 | 0.12 |
| Staphylococcus aureus | SSC 82-24 | 0.03 |
| Staphylococcus aureus | SSC 82-54 | 0.12 |
| Staphylococcus epidermidis | CMC 83-133 | 0.008 |
| Staphylococcus epidermidis | ATCC 12228 | 0.015 |
| Streptococcus faecalis | ATCC 29212 | 0.12 |
| Streptococcus faecalis | CMC 83-53 | 0.5 |
| Streptococcus faecalis | IO 83-28 | 0.12 |

This pseudoaglycone is also active as an antitumor agent as determined in the Biochemical Induction Assay (BIA), a bacterial assay system which specifically measures the ability of an agent to directly or indirectly initiate DNA damage. The indicator organism for this test is an *E. coli-lambda* lysogen, genetically constructed such that a DNA damaging event results in the expression of the gene for the enzyme β-galactosidase. This enzyme can be determined qualitatively or quantitatively by biochemical assay as an indication that DNA damage has occurred.

A modified version of the quantitative liquid BIA disclosed by Elespuru, R. and Yarmolinsky, M., Environmental Mutagenesis, 1, 65 (1979) was used to evaluate these compounds.

The antitumor activity of the pseudoaglycone of LL-E33288 was further demonstrated in the following test.

### Lymphocytic leukemia P388 test

The animals used were BDF1 mice, all of one sex, weighing a minimum of 17 g and all within a 3 g weight range. There were 5 or 6 animals per test group. The tumor transplant was by intraperitoneal injection of 0.5 ml of dilute ascitic fluid containing 10⁶ cells of lymphocytic leukemia P388. The test compounds were administered intraperitoneally at the indicated doses at a volume of 0.5 ml in 0.2% Klucel in normal saline on days 1, 5 and 9, relative to tumor inoculation. The animals were weighed and survivors recorded on a regular basis for 30 days. The percent increase in life span was calculated from the ratio of survival time for treated/control mice. The results appear in Table IV.

**TABLE IV**

| Lymphocytic Leukemia P388 Test | | |
|---|---|---|
| Compound | Dose (mg/kg) | % Increased Life Span |
| Pseudoaglycone of LL-E33288 | 200 | 164 |
| | 160 | 178 |
| | 80 | 157 |
| | 40 | 154 |
| | 20 | 154 |
| | 10 | 146 |
| | 5 | 125 |
| | 2.5 | 127 |
| | 1.2 | 114 |

### Example 10

### Preparation of the Pseudoaglycone of LL-E33288

A methanolic solution of partially purified LL-E33288γ₁-I (408 mg, 65% pure, in 5 ml) was passed slowly through a column (1.5 x 30cm) packed with Dowex® 50W-X8 (50-100 mesh, hydrogen form) prewashed with dichloromethane and methanol and equilibrated with methanol. The column effluent was monitored by TLC (Whatman LHP-KP Linear-K high performance silica gel precoated glass plates, ethyl acetate saturated with 0.1M phosphate buffered at pH 7 elution, detected by UV₂₅₄ₙₘ quenching and charring after spraying with a solution of #5 cupric acetate in 8% aqueous phosphoric acid) and was recycled back onto the column until no γ₁-I was detected. The column was eluted with 4 liters of methanol overnight, the eluate was collected and concentrated in vacuo to dryness. The light yellow residue was triturated with t-butyl methyl ether and the insolubles were redissolved in ethyl acetate and precipitated by addition of hexane to yield 121 mg of 82% pure pseudoaglycone of LL-E33288.

The 82% pure pseudoaglycone of LL-E33288 was further purified by chromatography on a Bio-Sil® A (20-44µ) column (0.9 x 25cm) eluting with dichloromethane:methanol (95:5) to yield 73 mg of analytically pure pseudoaglycone of LL-33288.

### Example 11

### Preparation of triphenylphosphine reaction product of the pseudoaglycone of LL-33288

A 279 mg sample of 80% pure pseudoaglycone of LL-E33288 was dissolved in a mixture of 40 ml of dichloromethane and 20 ml of methanol. A 140 mg portion of triphenylphosphine was added and the reaction mixture was stirred under argon for 3 hours. The mixture was concentrated in vacuo to dryness. The residue was redissolved in ethyl acetate and precipitated by the addition of hexane. The precipitate was chromatographed on a Woelm silica (32-63µ) column, eluting with dichloromethane:methanol (95:5). Fractions containing the desired material [Rf 0.23, Whatman LHP-KF Linear-K high performance silica gel precoated glass plates, dichloromethane:methanol (94:6) elution] were pooled and worked up by concentration and precipitation to yield 67 mg of 90% pure triphenylphosphine reaction product of the pseudoaglycone of LL-E33288.

The 90% pure triphenylphosphine reaction product of the pseudoaglycone of LL-33288 was further purified by preparative TLC on two Analtech, 20 x 20cm, 2000µ layer, silica gel GF precoated plates, eluting with ethyl acetate saturated with 0.1M phosphate buffer at pH 7. The major UV₂₅₄ₙₘ quenching band (Rf 0.4) was worked up to yield 49 mg of analytically pure triphenylphosphine reaction product of the pseudoaglycone of LL-E33288.

### Example 12

### Preparation of the reaction product of the product, derived from reacting the pseudoaglycone of LL-E33288 with triphenylphosphine, with methanolic potassium carbonate and then excess acetic anhydride

A 46 mg sample of 90% triphenylphosphine reaction product of the pseudoaglycone of LL-E33288 was dissolved in 4 ml of methanol and 4 ml of a saturated methanolic solution of potassium carbonate was added. The reaction mixture was allowed to remain at room temperature for 5 minutes, then was treated with 400 µl of acetic anhydride and allowed to remain at 4°C for 2 hours. After neutralization with methanolic potassium carbonate, the reaction mixture was concentrated in vacuo to dryness. The residue was chromatographed on two Analtech, 20 x 20cm, 2000µ layer, silica gel GF precoated plates, eluting with dichloromethane:methanol (94:6). The two major UV₂₅₄ₙₘ quenching, UV₃₆₆ₙₘ blue fluorescent bands, chromatographing close to each other, were worked up together and the mixture was rechromatographed on four Analtech, 20 x 20cm, 1000µ layer, silica gel GF precoated plates, eluting with the same solvent system to yield 7.5 mg of crystalline compound, which was recrystallized from a mixture of methanol and chloroform to give crystals suitable for x-ray crystallography.

### EXAMPLE 13

### Preparation of Dihydro-LL-E33288γ₁-I

A 10 ml portion of methyl iodide was added to a solution of 126 mg of LL-E33288γ₁-I in 25 ml of ethanol and the mixture was cooled in an ice-water bath. To this was added 12 ml of a 0.1M ethanolic solution of sodium borohydride, in 2 ml portions. When the reaction was complete, the borate complex was decomposed by the addition of 1.2 ml of a 4M ethanolic solution of acetic acid. The reaction mixture was then concentrated to a golden yellow residue which was redissolved in ethyl acetate and then reconcentrated to dryness. This residue was redissolved in ethyl acetate, the insolubles filtered off, the filtrate concentrated to a small volume and precipitated by the addition of hexane. The 301 mg of crude dihydro-LL-E33288γ₁-I was purified by chromatography on a Bio-Sil A (20-44µ) column, eluting with dichloromethane:methanol (92:8), giving 57 mg of pure dihydro-LL-E33288γ₁-I as a 30:70 mixture of two regio isomers.

### Example 14

### Preparation of Dihydro-LL-E33288-pseudoaglycone

A 10 ml portion of methyl iodide was added to a solution of 112 mg of LL-E33288-pseudoaglycone in 25 ml of ethanol and this mixture was cooled in an ice-water bath. To this was added 12 ml of a 0.025M ethanolic sodium borohydride in 2 ml portions. When the reaction was complete, the borate complex was decomposed by the addition of 1.2 ml of a 1M ethanolic solution of acetic acid. The reaction mixture was then concentrated to a golden yellow residue, redissolved in ethyl acetate and then reconcentrated to dryness. This residue was redissolved in ethyl acetate, the insolubles filtered off, the filtrate concentrated to a small volume and precipitated by the addition of hexane. The 128 mg of crude dihydro-LL-E33288-pseudo-aglycone was purified by chromatography on a Bio-Sil A (20-44µ) column, eluting with dichloromethane:methanol (97:3), giving 42 mg of pure dihydro-LL-E33288-pseudo-aglycone.

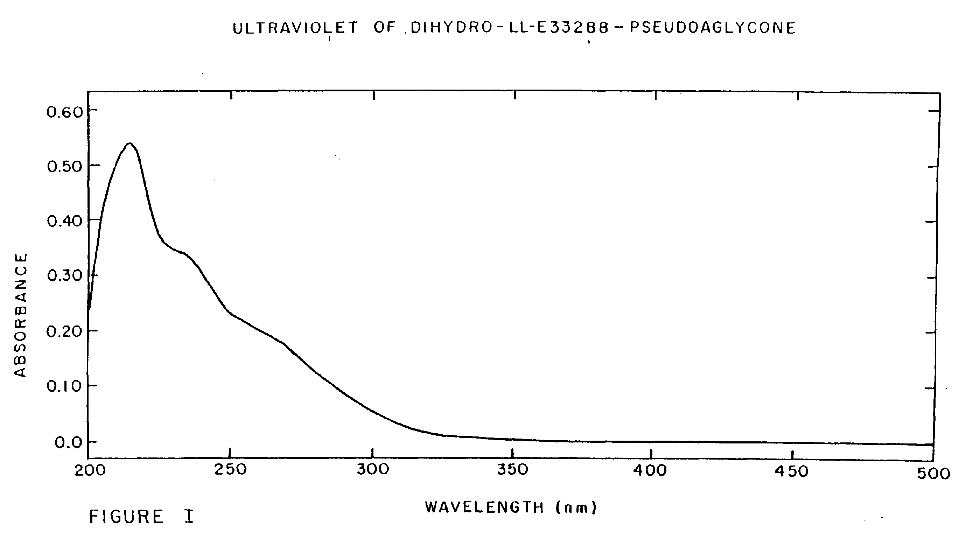

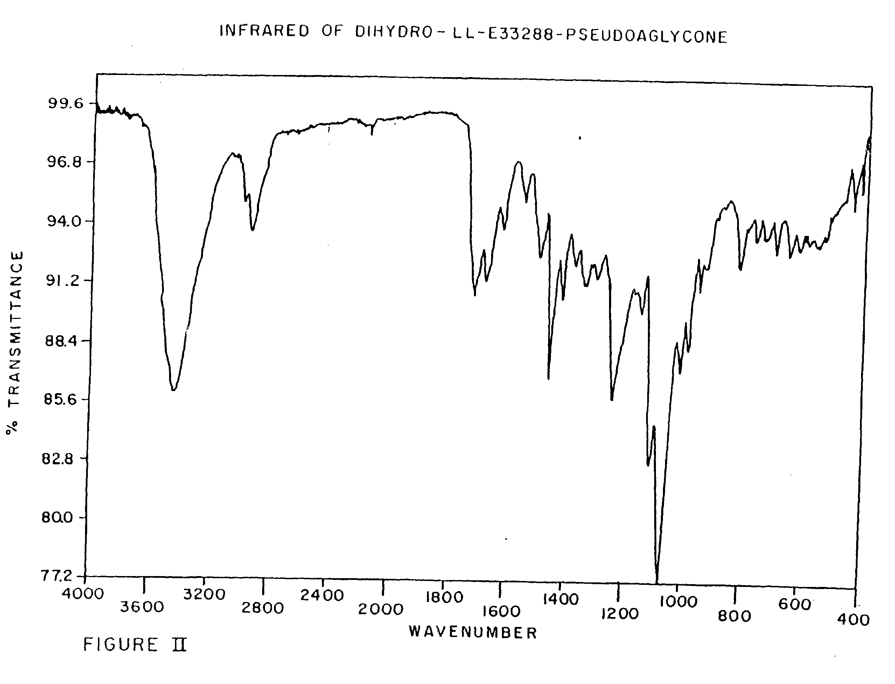

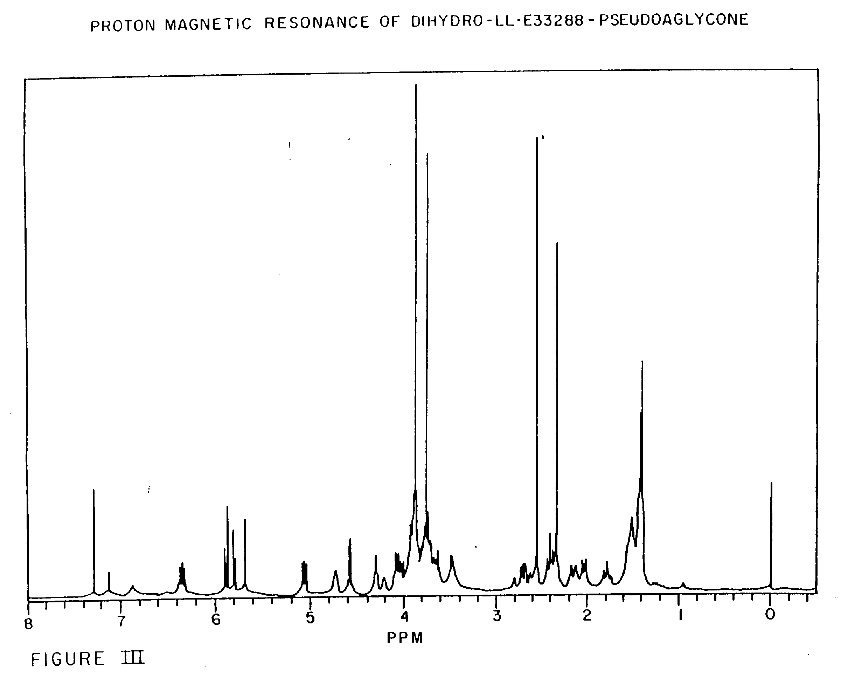

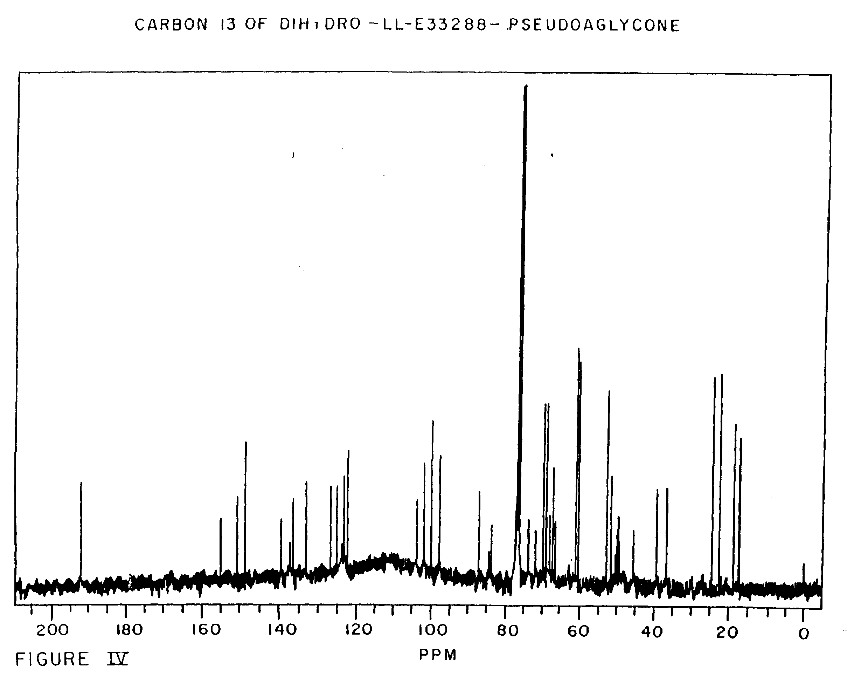

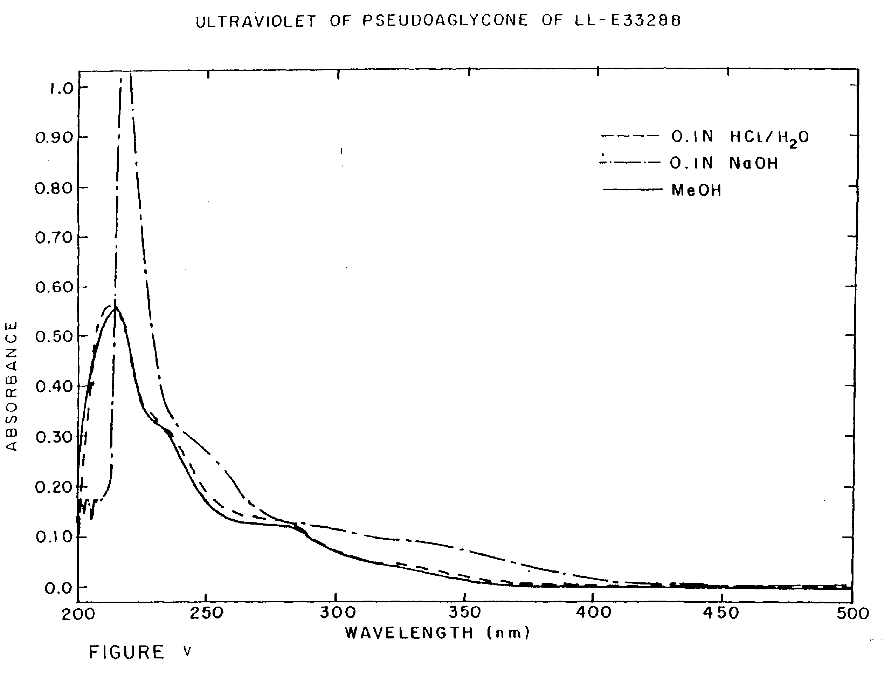

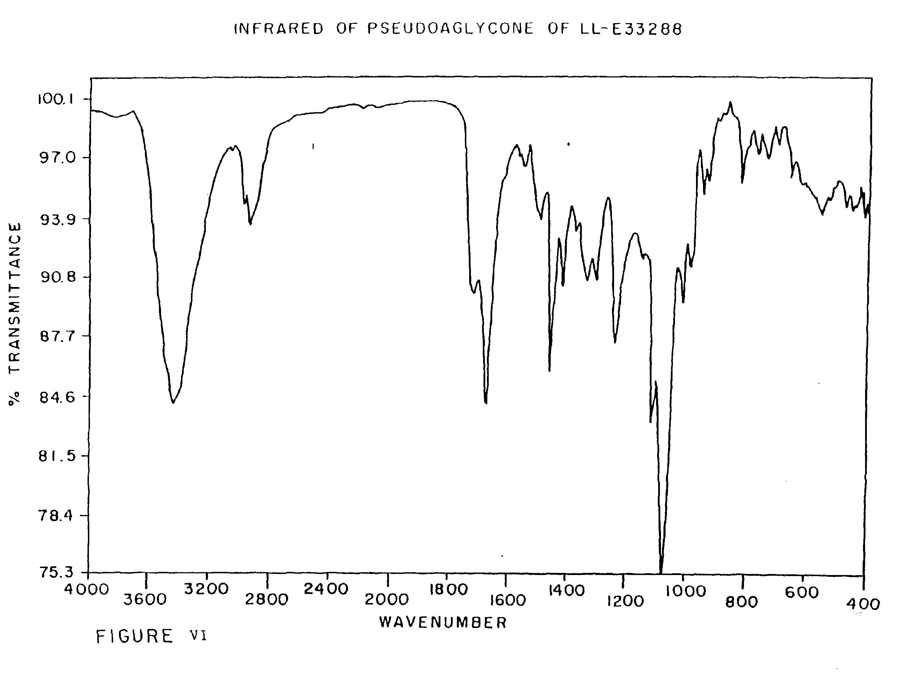

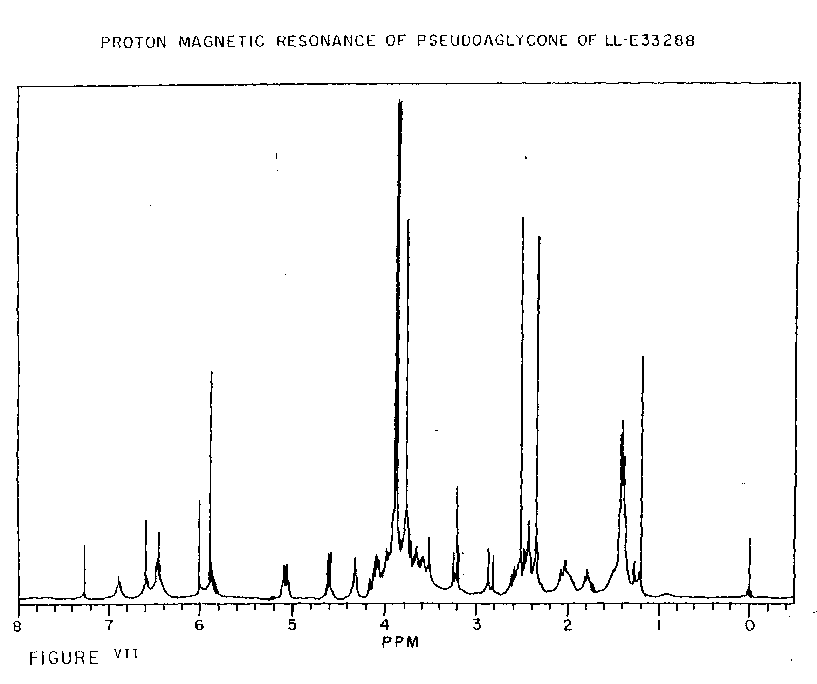

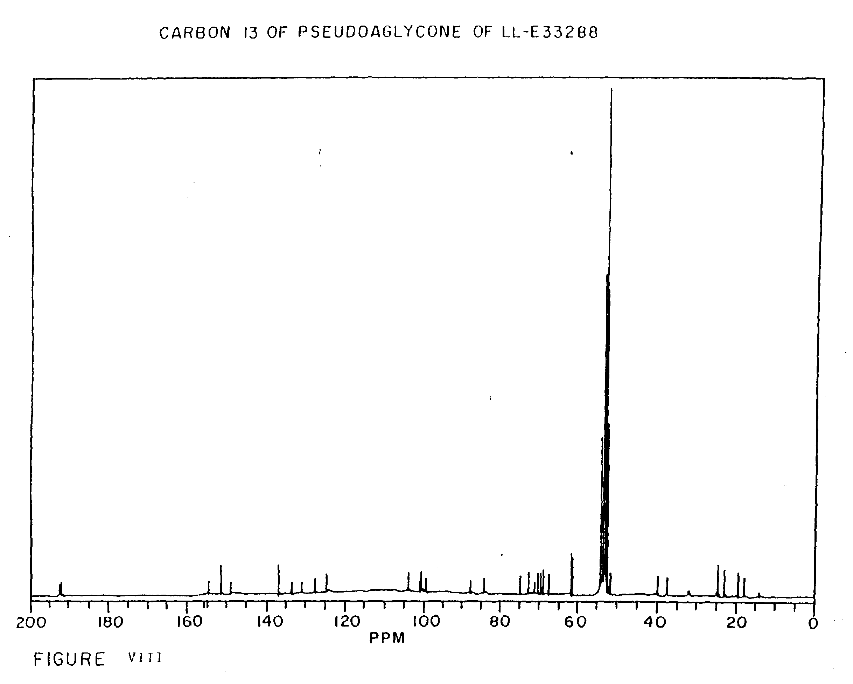

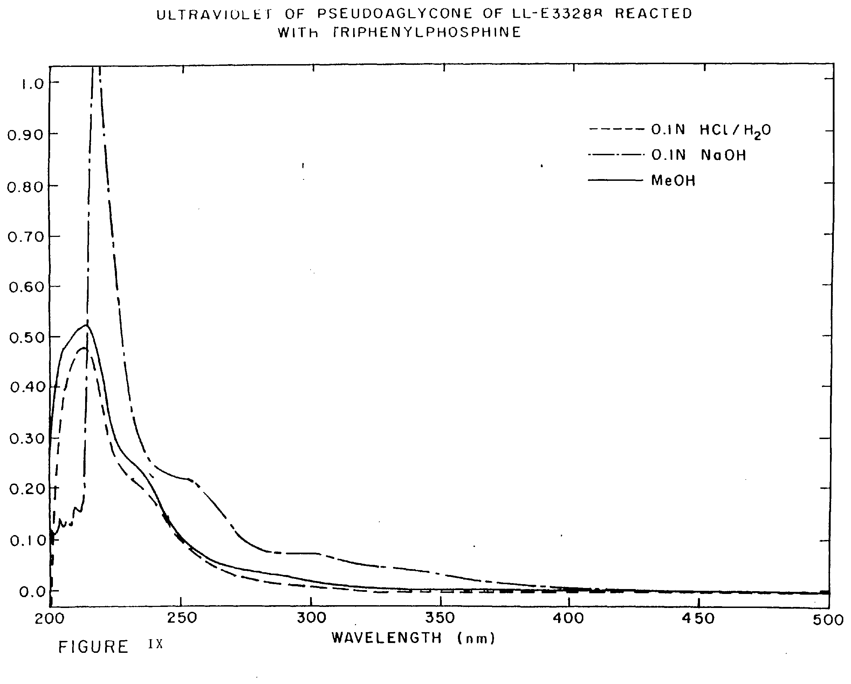

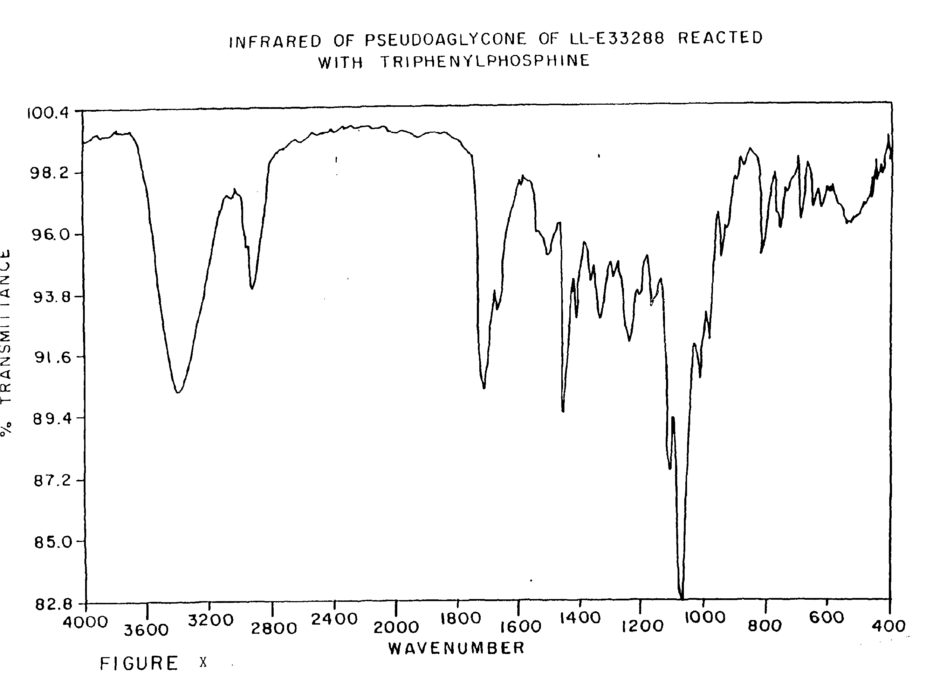

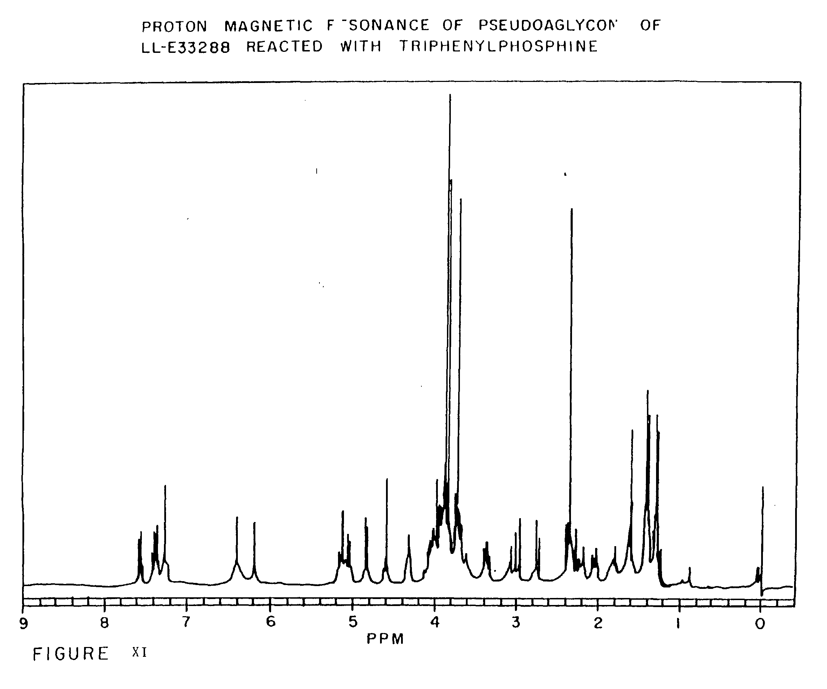

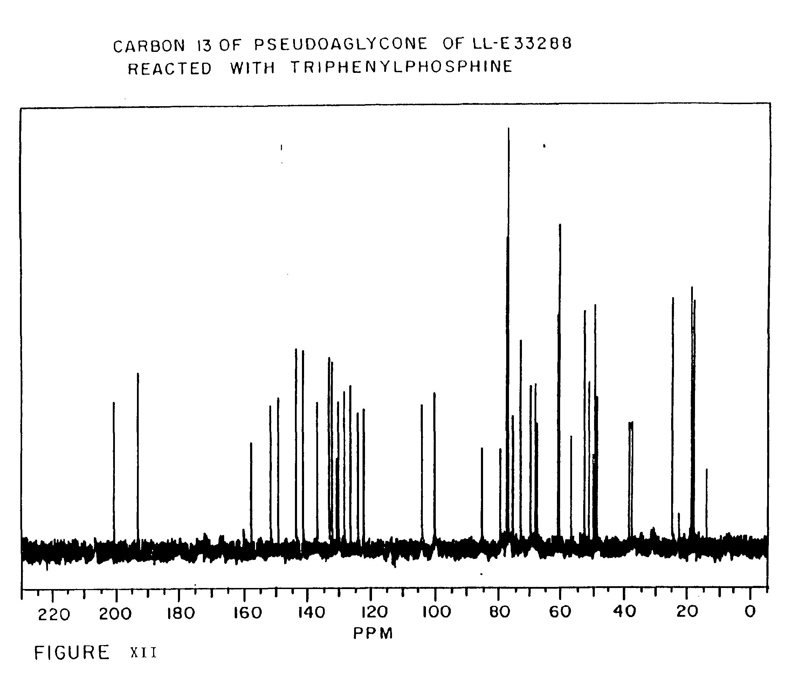

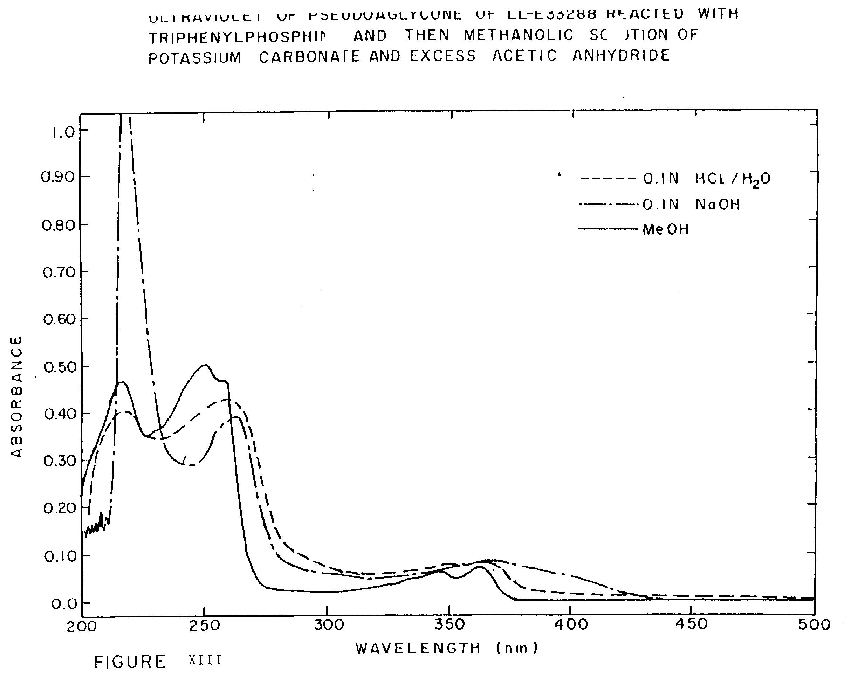

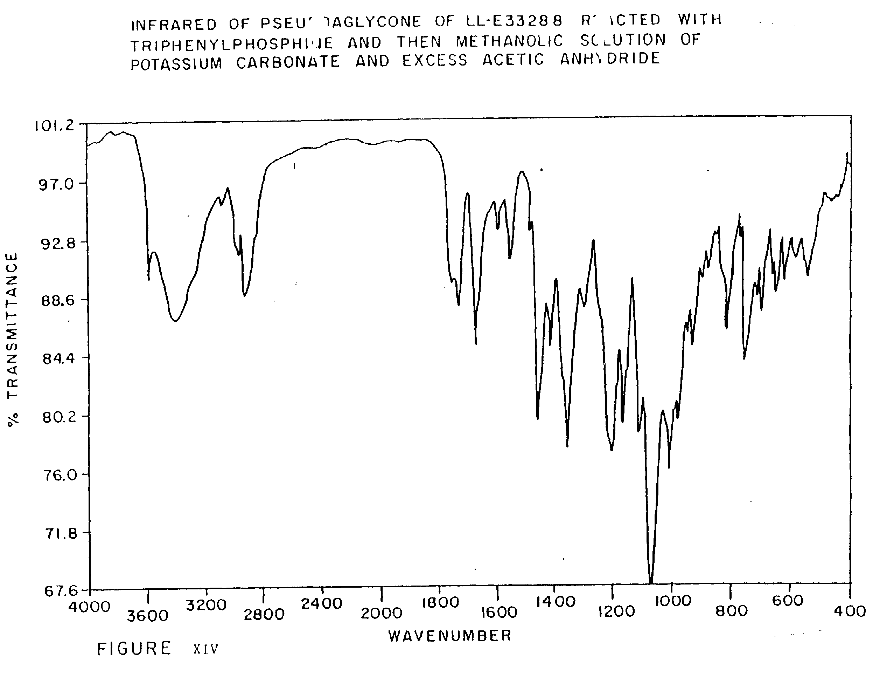

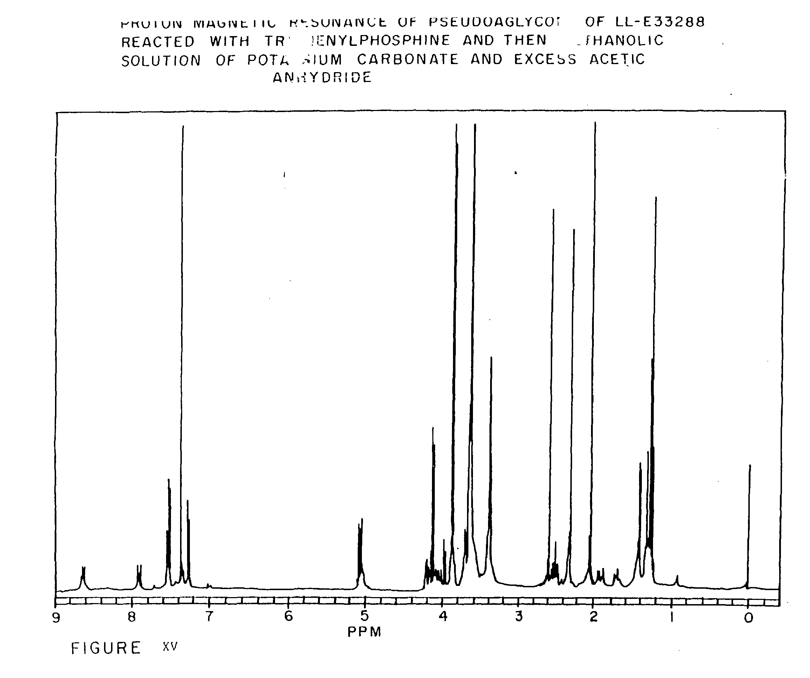

## Claims

1. The iodinated pseudoaglycone of LL-E33288 and its dihydro derivative.

2. The brominated pseudoaglycone of LL-E33288 and its dihydro derivative.

3. The dihydro derivative of LL-E33288α₁-Br, LL-E33288α₂-Br, LL-E33288α₃-Br, LL-E33288α₄-Br, LL-E33288β₁-Br, LL-E33288β₂-Br, LL-E33288γ₁-Br, LL-E33288α₁-I, LL-E33288α₂-I, LL-E33288α₃-I, LL-E33288β₁-I, LL-E33288β₂-I, LL-E33288γ₁-I, and LL-E33288δ₁-I.

4. A compound dihydro-BBM-1675; dihydro-FR-900405; dihydro-FR-900406; dihydro-PD-114759; dihydro-PD-115028; dihydro-CL-1577A; dihydro-CL-1577B; dihydro-CL-1577D; dihydro-CL-1577E; or dihydro-CL-1724.

5. A compound dihydro-BBM-1675; dihydro-FR-900405; dihydro-FR-900406; dihydro-PD-114759; dihydro-PD-115028; dihydro-CL-1577A; dihydro-CL-1577B; dihydro-CL-1577D; dihydro-CL-1577E; or dihydro-CL-1724 prepared by treating a solution of BBM-1675; FR-900405; FR-900406; PD-114759; PD-115028; CL-1577A; CL-1577B; CL-1577D; CL-1577E or CL-1724 in ethanol and an alkyl iodide with sodium borohydride at 0°C, decomposing with acetic acid, precipitating from ethyl acetate solution with hexane and purification by chromatography, giving dihydro-BBM-1675; dihydro-FR-900405; dihydro-FR-900406; dihydro-PD-114759; dihydro-PD-115028; dihydro-CL-1577A; dihydro-CL-1577B; dihydro-CL-1577D; dihydro-CL-1577E or dihydro-CL-1724.

6. A compound pseudoaglycone of BBM-1675; FR-900405; FR-900406; PD-114759; PD-115028; CL-1577A; CL-1377B; CL-1577D; CL-1577E or CL-1524.

7. A method of treating bacterial infections in warm-blooded animals which comprises administering to said animals an antibacterially effective amount of a compound of the iodinated or brominated pseudoaglycone of LL-E33288 or their dihydro derivatives; or the dihydro derivatives of LL-E33288α₁-Br; LL-E33288α₁-I; LL-E33288α₂-Br; LL-E33288α₂-I; LL-E33288α₃-Br; LL-E33288α₃-I; LL-E33288α₄-Br; LL-E33288β₁-Br; LL-E33288β₁-I; LL-E33288β₂-Br; LL-E33288β₂-I; LL-E33288γ₁-Br; LL-E33288γ₁-I; or LL-E33288δ₁-I.

8. A method of inhibiting the growth of tumors in a mammal comprising administering to said mammal an effective amount of a compound of the iodinated or brominated pseudoaglycone of **LL-E33288** or their dihydro derivatives; or the dihydro derivatives of LL-E33288α₁-Br; LL-E33288α₁-I; LL-E33288α₂-Br; LL-E33288α₂-I; LL-E33288α₃-Br; LL-E33288α₃-I; LL-E33288α₄-Br; LL-E33288β₁-Br; LL-E33288β₁-I; LL-E33288β₂-Br; LL-E33288β₂-I; LL-E33288γ₁-Br; LL-E33288γ₁-I; or LL-E33288δ₁-I.

9. A method of regressing leukemia in a mammal comprising administering to said mammal an effective amount of a compound of the iodinated or brominated pseudoaglycone of LL-E33288 or their dihydro derivatives; or the dihydro derivatives of LL-E33288α₁-Br; LL-E33288α₁-I; LL-E33288α₂-Br; LL-E33288α₂-I; LL-E33288α₃-Br; LL-E33288α₃-I; LL-E33288α₄-Br; LL-E33288β₁-Br; LL-E33288β₁-I; LL-E33288β₂-Br; LL-E33288β₂-I; LL-E33288γ₁-Br; LL-E33288γ₁-I; or LL-E33288δ₁-I.

10. A process for producing antibiotics LL-E33288α₁-Br; LL-E33288α₂-Br; LL-E33288α₃-Br; LL-E33288α₄-Br; LL-E33288β₁-Br; LL-E33288β₂-Br; and LL-E33288γ₁-Br which comprises aerobically fermenting the organism Micromonospora echinospora ssp. calichensis mutant NRRL-18149 of NRRL-15839 or mutants of NRRL-18149 in a liquid medium containing assimilable sources of carbon, nitrogen, bromine and inorganic salts, until substantial antibiotic activity is imparted to said medium and then recovering the antibiotics therefrom.

11. A process for producing antibiotics LL-E33288α₁-Br; LL-E33288α₂-Br; LL-E33288α₃-Br; LL-E33288α₄-Br; LL-E33288β₁-Br; LL-E33288β₂-Br; and LL-E33288γ₁-Br which comprises aerobically fermenting a liquid medium containing assimilable sources of carbon, nitrogen, bromine and inorganic salts; which medium has been inoculated with a viable culture of the organism Micromonospora echinospora ssp. calichensis mutant NRRL-18149 of NRRL-15839 or mutants of NRRL-18149, maintaining said fermentation culture at a temperature of about 24-32°C for a period of approximately 90-200 hours, harvesting the mash and extracting the antibiotics.

12. A process for producing antibiotics LL-E33288α₁-I; LL-E33288α₂-I; LL-E33288α₃-I; LL-E33288β₁-I; LL-E33288β₂-I; LL-E33288γ₁-I; and LL-E33288δ₁-I which comprises aerobically fermenting the organism Micromonospora echinospora ssp. calichensis mutant NRRL-18149 of NRRL-15839 or mutants of NRRL-18149 in a liquid medium containing assimilable sources of carbon, nitrogen, iodine and inorganic salts, until substantial antibiotic activity is imparted to said medium and then recovering the antibiotics therefrom.

13. A process for producing antibiotics LL-E33288α₁-I; LL-E33288α₂-I; LL-E33288α₃-I; LL-E33288β₁-I; LL-E33288β₂-I; LL-E33288γ₁-I and LL-E33288δ₁-I which comprises aerobically fermenting a liquid medium containing assimilable sources of carbon, nitrogen, iodine and inorganic salts; which medium has been inoculated with a viable culture of the organism Micromonospora echinospora ssp. calichensis mutant NRRL-18149 of NRRL-15839 or mutants of NRRL-18149, maintaining said fermentation culture at a temperature of about 24-32°C for a period of approximately 90-200 hours, harvesting the mash and extracting the antibiotics. antibiotic activity is imparted to said medium and then recovering the antibiotics therefrom.

14. A process for producing the compound dihydro-LL-E33288 α₁-Br; dihydro-LL-E33288α₁-I; dihydro-LL-E33288 α₂-Br; dihydro-LL-E33288 α₂-I; dihydro-LL-E33288 α₃-Br; dihydro-LL-E33288 α₃-I; dihydro-LL-E33288 α₄-Br; dihydro-LL-E33288 β₁-Br; dihydro-LL-E33288 β₁-I; dihydro-LL-E33288 β₂-Br; dihydro-LL-E33288 β₂-I; dihydro-LL-E33288 γ₁-Br; dihydro-LL-E33288 γ₁-I; dihydro-LL-E33288δ₁-I; dihydro-BBM-1675; dihydro-FR-900405; dihydro-FR-900406; dihydro-PD-114759; dihydro-PD-115028; dihydro-CL-1577A; dihydro-CL-1577B; dihydro-CL-1577E; or dihydro-CL-1724 which comprises adding an alkyl iodide to an ethanolic solution of the antibiotic, cooling the solution to Ice-bath temperature, adding an ethanolic solution of sodium borohydride, decomposing the borate complex by the addition of acetic acid, precipitating from an ethyl acetate solution by the addition of hexane and purifying by chromatography.

15. A process for preparing the pseudoaglycone of an antibiotic of the LL-E33288 complex, BBM-1675, FR-900405, FR-900406, PD 114759, PD 115028, CL-1577A, CL-1577B, CL-1577D, CL-1577E or CL-1724 which comprises reacting a methanolic solution of the antibiotic with a cation exchange resin, followed by purifying by chromatography.

16. A culture containing the microorganism Micromonospora echinospora asp. calichensis NRRL-18149, said culture being capable of producing the LL-E33288 complex in recoverable quantity upon aerobic fermentation in an aqueous medium containing assimilable sources of carbon nitrogen, inorganic salts, and either iodine or bromine or both.

17. A composition of matter in dosage unit form comprising an antibacterially effective amount of a compound of Claims 1-6.
